Europäisches Patentamt

European Patent Office

Office européen des brevets

(11) Veröffentlichungsnummer: **0 295 618 B1**

# (12) EUROPÄISCHE PATENTSCHRIFT

(45) Veröffentlichungstag der Patentschrift: **11.12.91**

(51) Int. Cl.⁵: **A61F 5/01**

(21) Anmeldenummer: **88109429.6**

(22) Anmeldetag: **14.06.88**

(54) **Biegeelastisches Organ.**

(30) Priorität: **15.06.87 DE 3719960**

(43) Veröffentlichungstag der Anmeldung:
**21.12.88 Patentblatt 88/51**

(45) Bekanntmachung des Hinweises auf die
Patenterteilung:
**11.12.91 Patentblatt 91/50**

(84) Benannte Vertragsstaaten:
**CH FR GB IT LI NL SE**

(56) Entgegenhaltungen:
**DE-A- 3 245 471**
**DE-A- 3 508 844**
**DE-C- 124 746**

(73) Patentinhaber: **WILHELM JULIUS TEUFEL GMBH**
**Neckarstrasse 189-191**
**W-7000 Stuttgart 1(DE)**

(72) Erfinder: **Bähler, André**
**Kreuzstr. 46**
**CH-8008 Zürich(CH)**
Erfinder: **Wolfer, Romano**
**Hohentwielstrasse 150**
**W-7000 Stuttgart 1(DE)**

(74) Vertreter: **Patentanwälte RUFF, BEIER und SCHÖNDORF**
**Neckarstrasse 50**
**W-7000 Stuttgart 1(DE)**

## Beschreibung

Die Erfindung betrifft ein biegeelastisches Organ für orthopädische Zwecke in Form einer Schraubenfeder nach dem Oberbegriff des Anspruchs 1.

Ein derartiges Organ in Form einer Hüftorthese für Säuglinge ist aus der (DE-A- 35 08 844) bekannt. Bei dieser Hüftorthese sind zwischen einem Beckengürtel und den Manschetten zur Aufnahme der Beine des Säuglings biegeelastische Organe in Form von Schraubenfedern angeordnet. Diese sollen es ermöglichen, daß der Säugling seine Beine aus einer vorgegebenen Stellung heraus gegen einen wachsenden Widerstand bewegen kann.

In vielen Fällen ist es jedoch erforderlich, daß zu Anfang der Behandlung eine starre Fixierung der Beine vorgenommen werden kann und daß die starre Fixierung im Laufe des Heilprozeßes in eine weniger starre Fixierung umgewandelt werden kann.

Ebenfalls bekannt ist ein Muskelstärker in Form einer Schraubenfeder (DE-C- 12 47 46), die von einem Drahtseil durchsetzt ist, und an deren Enden je ein Handgriff angebracht ist.

Bei starker Ausbiegung der Shraubenfeder werden durch das Drahtseil zusätzliche Federn gespannt, so daß der Widerstand, den der Muskelstärker dem Benutzer entgegengesetzt, vergrößert werden kann. Die Schraubenfeder bleibt immer verbiegbar.

Der Erfindung liegt die Aufgabe zugrunde, ein biegeelastisches Organ für orthopädische Zwecke, das beispielsweise bei Hüftorthesen für Säuglinge verwendet werden kann, zu schaffen, das einerseits starr und auderseits verbiegbar bzw. auslenkbar eingestellt werden kann.

Zur Lösung dieser Aufgabe schlägt die Erfindung vor, daß im Inneren der Schraubenfeder ein biege elastisches bzw. biegbares Band augeordnet ist, das im Bereich beider Enden der Shraubenfeder derart ausgebildet ist, daß es bei einer durch Biegen der Schraubenfeder bewirkten Verlängerung der Schraubenfeder zu einer das weitere Biegen der Schraubenfeder verhindernden Anlage an dem jeweiligen Ende der Schraubenfeder oder einem mit diesem verbundenen Teil gelangt, wobei der Abstand zwischen den aneinander zur Anlage gelangenden Teilen veränderbar bzw. einstellbar ist.

Auf diese Weise wird es möglich, die Verbiegbarkeit des Organs zwischen einer vollständigen Starrheit und einem beliebigen Auslenkungswinkel zu verstellen. Bei der Anwendung bei einer Hüftorthese kann das Organ zunächst bei Beginn der Behandlung starr gemacht und nach Einleitung der Bearbeitung durch einfache Verstellung des Abstandes nach und nach immer stärker verbiegbar gemacht werden bis beispielsweise zu einem Winkel von 70°. Die Rückstellkraft bleibt dabei erhalten.

Die Anlage im Bereich eines Schraubenfederendes kann beispielsweise dadurch geschehen, daß das Band an seinem einen Ende mit der Schraubenfeder direkt oder mit einem mit dieser verbundenen bzw. verbindbaren Teil verbunden ist. Beispielsweise kann das Band an dem einem Ende der Schraubenfeder direkt mit dieser verlötet werden.

Ebenfalls mit Vorteil möglich ist es, daß das Band an seinem einen Ende mit einem Element verbunden ist, dessen Querabmessung größer als die Querabmessung des Innenraums der Schraubenfeder ist. Auch dies führt zu einer Anlage.

Eine Möglichkeit der Verstellung des Abstands zwischen den aneinander zur Anlage gelangenden Teilen und damit zur Einstellung der maximalen Verbiegbarkeit liegt darin, daß das Band an seinem einen Ende an einem Gleitstück befestigt ist, das im Bereich des zugehörigen Schraubenfederendes verschiebbar angeordnet ist, wobei der Verschiebeweg einstellbar ist.

Eine besonders bevorzugte Möglichkeit der verschiebbaren Führung ist dann gegeben, wenn das Schraubenfederende mit einer Hülse verbunden ist, in der das Gleitstück verschiebbar geführt ist. Es kann dabei zwischen einer Schulter und einem gegenüber der Hülse beispielsweise durch ein Gewinde verdrehbaren Element geführt sein. Die Verwendung eines Gewindes macht eine stufenlose Verstellung besonders einfach möglich.

Besonders günstig ist es, wenn das Gleitstück ein Gewinde aufweist, das mit einem Gegengewinde eines Gegenelements zusammenwirkt, das mit einer Anlagefläche der Hülse zusammenwirkt. Diese Anlagefläche kann z. B. eine Stirnfläche der Hülse sein, aber auch eine Schulter an einer Durchmesserveränderung.

Insbesondere ist es günstig, wenn in Weiterbildung das Gleitstück einen Gewindezapfen aufweist und das Gegenelement eine Mutter, die an einer Schulter der Hülse zur Anlage gelangt. Die Schulter kann auch die Stirnfläche der Hülse sein.

Um sicherzustellen, daß die Verstellung nicht zufällig geschieht, kann erfindungsgemäß vorgesehen sein, daß die Mutter vollständig in der Hülse untergebracht ist, also nicht nach außen vorspringt. Zur Betätigung der Mutter kann diese an ihrem äußeren Ende eine Betätigungsvertiefung oder einen Betätigungsansatz aufweisen, beispielsweise eine Sechskantvertiefung. Dann läßt sich die Mutter nur mit Hilfe eines zugehörigen Schlüssels verstellen. Insbesondere kann die Mutter eine Selbsthemmung aufweisen.

In Weiterbildung der Erfindung kann vorgesehen sein, daß das Band das Innere der Schraubenfeder im wesentlichen ausfüllt, insbesondere in radialer Richtung. In diesem Fall ist also zwischen der Außenseite des Bandes und der Innenseite der Schraubenfeder kein oder nur ein geringer Abstand vorhanden.

Das biegeelastische Band kann mit Vorteil ein Drahtseil sein, das also eine große Anzahl von verseilten Drähten aufweist.

Insbesondere kann vorgesehen sein, daß benachbarte Windungen der nicht gebogenen Schraubenfeder aneinander anliegen.

Weitere Merkmale, Einzelheiten und Vorzüge der Erfindung ergeben sich aus der folgenden Beschreibung bevorzugter Ausführungsformen der Erfindung sowie anhand der Zeichnung. Hierbei zeigen:

Fig. 1    einen Längsschnitt durch eine erste Ausführungsform der zusammengebauten biegeelastischen Organs in starrer Stellung;

Fig. 2    einen Längsschnitt durch ein Einzelteil des Organs;

Fig. 3    eine Aufsicht auf das in Fig. 2 dargestellte Einzelteil;

Fig. 4    einen Längsschnitt durch ein weiteres Einzelteil des biegeelastischen Organs;

Fig. 5    einen Längsschnitt durch ein zweites biegeelastisches Organ in starrer Stellung.

Die Schraubenfeder 1 nimmt in sich ein biegeelastisches Band 2 auf, das z. B. ein aus Stahldrähten bestehendes Seil sein kann und das an seinem einen, in der Zeichnung unteren Ende mit der Schraubenfeder 1 verbunden, beispielsweise hartverlötet ist und das an seinem anderen, in der Zeichnung oberen Ende in den unteren, einen Rechteckquerschnitt aufweisenden napfförmigen Teil 32 eines Gleitstücks 3 eingreift, mit dem das Band 2 ebenfalls hartverlötet ist. Das Gleitstück 3 gleitet mit diesem Teil 32 in einer entsprechend ausgebildeten Führung einer Hülse 4, die an ihren beiden Enden über eine Teillänge einen größeren lichten Durchmesser aufweist. Diese Hülse 4 nimmt an ihrem unteren Ende die Schraubenfeder 1 in sich auf, mit der die Hülse 4 verlötet oder verschweißt ist. An ihrem oberen Ende nimmt die Hülse 4 die zapfenförmige Verlängerung 51 der Mutter 5 in sich auf, deren Kopf 52 einen größeren Durchmesser aufweist als die Hülse 4, so daß die Stirnfläche der Hülse 4 dem Kopf 52 der Mutter 5 als Anschlag dienen kann. Mit ihrem Innengewinde nimmt die zapfenförmige Verlängerung 51 der Mutter 5 den Gewindezapfen 31 des Gleitstücks 3 in sich auf, so daß bei gerader Erstreckung der Schraubenfeder 1 durch Verdrehen der Mutter 5 ein beliebiger Abstand des Kopfes 52 der Mutter 5 von der Stirnfläche der Hülse 4 eingestellt werden kann. Da bei einer Biegung der Schraubenfeder sich deren Längsachse verlängert, wird durch die Einstellung des vorgenannten Abstandes auch die Amplitude des Ausschlags einer einseitig eingespannten Schraubenfeder bzw. das Minimum des Krümmungsradius der abgebogenen Schraubenfeder 1 bestimmt. In das durchgehende Innengewinde der Mutter 5 ist eine Sicherungsschraube 6 eingeschraubt.

Insbesondere eignet sich ein solches biegeelastisches Organ für die Konstruktion orthopädischer Geräte, bei deren Anlegen an den Körper, z. B. nach einer Operation ein Körperglied anfänglich vollkommen stillgelegt und allmählich seine Bewegungsfreiheit unter Überwindung einer zunehmenden Gegenkraft vergrößert werden soll. Insbesondere als Glied einer Hüftgelenksorthese für Säuglinge ist ein solches biegeelastisches Organ geeignet.

Das erfindungsgemäße biegeelastische Organ kann in vereinfachter Weise auch so ausgebildet sein, daß das von der Schraubenfeder 1 umschlossene, an seinem einen Ende mit dem Ende der Schraubenfeder 1 verbundene Teil 2, z. B. ein biegeelastischer Stab, an seinem anderen über die Schraubenfeder 1 überstehenden Ende ein Gewinde aufweist, auf das eine über den Durchmesser der Schraubenfeder 1 überstehende Mutter 5 aufgeschraubt ist.

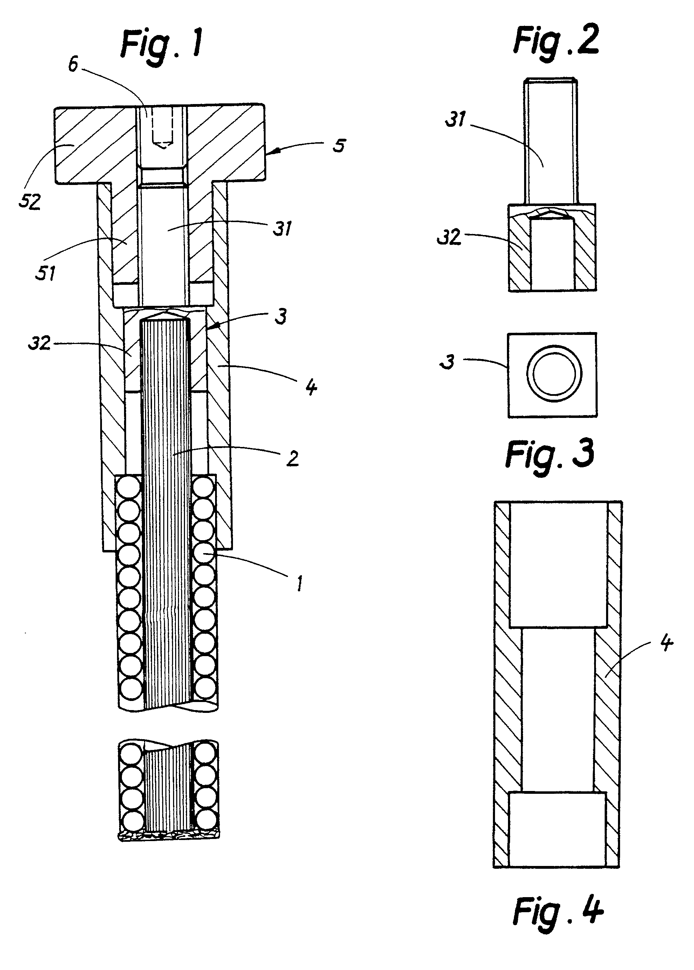

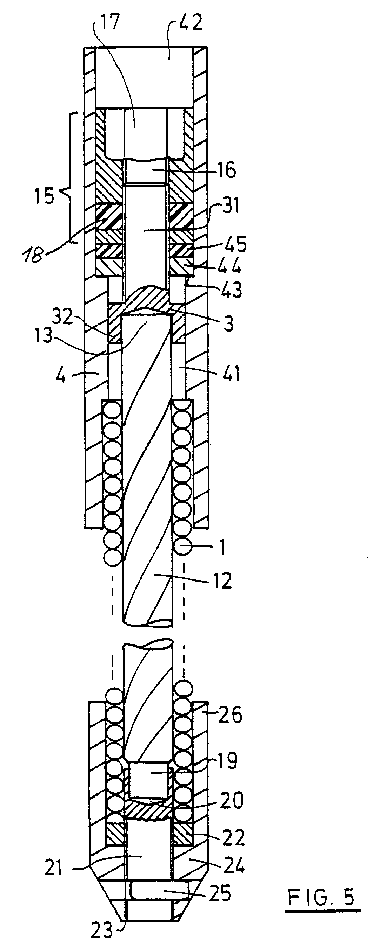

Bei der Ausführungsform der Fig. 5 ist die Befestigung der Schraubenfeder 1 an der Hülse in gleicher Weise realisiert wie bei der Ausführungsform der Fig. 1 - 4. Das hier verwendete biegeelastische Band 12 ist als Drahtseil ausgebildet und enthält eine Vielzahl von verdrallten Stahldrähten. Sein eines Ende 13 ist in gleicher Weise wie bei der Ausführungsform nach Fig. 1 in den napfförmigen Teil 32 des Gleitstücks 3 eingesetzt und mit diesem verbunden, beispielsweise durch Hartlöten.

Das Gleitstück 3 ist dadurch verdrehsicher in der Hülse 4 geführt, daß das Gleitstück 3 im Querschnitt ebenso wie der entsprechende Teil der Hülse 4 quadratisch ausgebildet ist. An dem Übergang zwischen dem im Querschnitt quadratischen Teil 41 des Innenraums der Hülse 4 und dem zum freien Ende des biegeelastischen Organs hin gerichteten Innenraum 42 mit dem vergrößerten Kreisdurchmesser ist eine Schulter 43 gebildet. Auf den Gewindezapfen 31 ist unter Zwischenlage von zwei Scheiben 44 und 45 eine Verstellmutter 15 aufgeschraubt, die eine mit einem Gewinde versehene Längsbohrung 16 aufweist. Im Bereich ihres äußeren Endes ist die Mutter 15 mit einer im Querschnitt sechseckigen Betätigungsvertiefung 17 versehen, in die ein Sechskantschlüssel eingesetzt werden kann. Die Mutter 15 weist einen Zwischenabschnitt 18 aus einem Kunststoffmaterial auf, der eine Verdrehsicherung bildet. Es handelt sich also um eine selbsthemmende Mutter, die sich nicht von selbst lösen kann.

Wie sich aus Fig. 5 ohne weiteres ergibt, ist die Mutter 15 vollständig im Inneren der Hülse 4 untergebracht, so daß sie nicht nach außen vorspringt und auch nicht von außen verstellt werden kann. Zu ihrer Verstellung ist ein Schlüssel erforderlich.

Am gegenüberliegenden Ende 19 des biegeelastischen Bandes 12 ist dieses in ähnlicher Weise in eine Ausnehmung 20 in einem Gewindestift 21 eingesetzt und dort verlötet. Auf den Gewindestift 20 ist eine Sechskantmutter 22 aufgeschraubt. Der Gewindestift 21 erstreckt sich durch eine Längsbohrung 23 einer Hülse 24 und ist in der dargestellten Stellung durch einen Querzapfen 25 gesichert. Der Querzapfen erstreckt sich durch eine Querbohrung des Gewindestifts 21 und eine fluchtende Querbohrung der Hülse 24.

In den radialen ringförmigen Zwischenraum zwischen der Außenseite des biegeelastischen Bandes 12 und der Innenseite der Wand 26 der Hülse 24 greift das eine Ende der Schraubenfeder 1 ein. Diese Schraubenfeder 1 kann mit der Hülse 24 fest verbunden sein, bei entsprechender Dimensionierung reicht es jedoch auch aus, wenn die Schraubenfeder nur in den Zwischenraum eingreift. Dieses Eingreifen kann ausreichen, wenn die Länge des in der Hülse 24 untergebrachten Abschnittes der Schraubenfeder 1 größer ist als der maximale Verstellweg der Mutter 15 auf dem Gewindezapfen 31.

In der dargestellten Stellung liegt die Unterseite der Mutter 15 unter Zwischenlage der beiden Scheiben 44, 45 an der Schulter 43 der Hülse 4 an. Eine Verbiegung des biegeelastischen Organs ist nicht möglich, da bei einer Verbiegung eine Längenänderung in der Mitte der Schraubenfeder 1 auftreten würde, was durch das biegeelastische Band 12 mit seinen Anschlägen verhindert wird. Wird die Mutter 15 etwas von der Schulter 43 weggeschraubt, läßt sich entsprechend das biegeelastische Organ etwas verbiegen, bis wieder eine Anlage erfolgt. Auf diese Weise läßt sich mit Hilfe der Mutter 15 eine stufenlose Verbiegbarkeit erreichen.

Der Widerstand, den das biegeelastische Organ einer Biegung entgegenstellt, wird im wesentlichen durch die Stärke und den Elastizitätsmodul des verwendeten Drahtes, den Durchmesser oder die Ganghöhe der einzelnen Windungen bestimmt. Natürlich kann auch eine gewisse Eigensteifigkeit des biegeelastischen Bandes 2 bzw. 12 hierzu beitragen. Das biegeelastische Band, das im wesentlichen undehnbar ist, dient hauptsächlich dazu, den Grad an Auslenkung zu begrenzen.

**Patentansprüche**

1. Biegeelastisches Organ für orthopädische Zwecke in Form einer Schraubenfeder (1) , dadurch gekennzeichnet, daß im Inneren der Schraubenfeder (1) ein biegeelastisches bzw. biegbares Band (2,12) angeordnet ist, das im Bereich beider Enden der Schraubenfeder (1) derart ausgebildet ist, daß es bei einer durch Biegen der Schraubenfeder (1) bewirkten Verlängerung der Schraubenfeder (1) zu einer das weitere Biegen der Schraubenfeder (1) verhindernden Anlage an dem jeweiligen Ende der Schraubenfeder (1) oder einem mit diesem verbundenen Teil (4, 24) gelangt, wobei der Abstand zwischen den aneinander zur Anlage gelangenden Teilen einstellbar ist.

2. Organ nach Anspruch 1, dadurch gekennzeichnet, daß das Band (2, 12) an seinem einen Ende (19) mit der Schraubenfeder (1) oder mit einem mit dieser verbundenen bzw. verbindbaren Teil (24) verbunden ist.

3. Organ nach Anspruch 1 oder 2, dadurch gekennzeichnet, daß das Band (2, 12) an seinem einen Ende (13, 19) mit einem Element (4, 24) verbunden ist, dessen Querabmessung größer als die Querabmessung des Innenraums der Schraubenfeder (1) ist.

4. Organ nach einem der vorhergehenden Ansprüche, dadurch gekennzeichnet, daß das Band (2,12) an seinem einen Ende an einem Gleitstück (3) befestigt ist, das im Bereich des zugehörigen Schraubenfederendes verschiebbar angeordnet ist, wobei der Verschiebeweg einstellbar ist.

5. Organ nach Anspruch 4, dadurch gekennzeichnet, daß das Gleitstück (3) in einer mit der Schraubenfeder (1) verbundenen Hülse 4) verschiebbar geführt ist.

6. Organ nach Anspruch 5, dadurch gekennzeichnet, daß das Gleitstück (3) ein Gewinde aufweist, das mit einem Gegengewinde eines Gegenelementes zusammenwirkt, das seinerseits mit einer Anlagefläche der Hülse (4) zusammenwirkt.

7. Organ nach einem der Ansprüche 4 bis 6, dadurch gekennzeichnet, daß das Gleitstück (3) einen Gewindezapfen (31) aufweist und das Gegenelement eine Mutter (5, 15) ist, die an einer Schulter (43) der Hülse 4 zur Anlage gelangt.

8. Organ nach Anspruch 7, dadurch gekennzeichnet, daß die Mutter (15) vollständig in der Hülse (4) untergebracht ist.

9. Organ nach Anspruch 7 oder 8, dadurch gekennzeichnet, daß die Mutter (5, 15) an ihrem äußeren Ende ein Betätigungselement, insbesondere eine Betätigungsvertiefung (17) aufweist.

10. Organ nach einem der vorhergehenden Ansprüche, dadurch gekennzeichnet, daß das Band (2, 12) das Innere der Schraubenfeder (1) im wesentlichen ausfüllt.

11. Organ nach einem der vorhergehenden Ansprüche, dadurch gekennzeichnet, daß das Band (2, 12) ein aus Stahldrähten bestehendes Drahtseil ist.

12. Organ nach einem der vorhergehenden Ansprüche, dadurch gekennzeichnet, daß benachbarten Windungen der ungebogenen Schraubenfeder (1) aneinander anliegen.

13. Organ nach einem der Ansprüche 4 - 12, dadurch gekennzeichnet, daß das Gleitstück (3) verdrehsicher angeordnet ist.

## Claims

1. Bending-elastic member for orthopedic purposes in the form of a helical spring (1), characterized in that within the helical spring (1) is provided a bending-elastic or bendable strip (2, 12), which is so formed in the vicinity of the two ends of the helical spring (1), that in the case of an extension of said spring caused by a bending thereof it engages on the particular end of the helical spring (1) or a part (4, 24) connected thereto so as to prevent further bending of the said spring, the distance between the parts engaging on one another being adjustable.

2. Member according to claim 1, characterized in that the strip (2, 12) is connected at its one end (19) to the helical spring (1) or to a part (24) connected or connectable thereto.

3. Member according to claims 1 or 2, characterized in that one end (13, 19) of the strip (2, 12) is connected to an element (4, 24), whose transverse dimension is larger than the transverse dimension of the interior of the helical spring (1).

4. Member according to one of the preceding claims, characterized in that the strip (2, 12) is fixed at its one end to a slider (3), which is displaceably located in the vicinity of the associated helical spring end, the displacement path being adjustable.

5. Member according to claim 4, characterised in that the slider (3) is displaceably guided in a sleeve (4) connected to the helical spring (1).

6. Member according to claim 5, characterised in that the slider (3) has a thread, which cooperates with a counterthread of a counterelement, which in turn cooperates with a contact surface of the sleeve (4).

7. Member according to one of the claims 4 to 6, characterized in that the slider (3) has a threaded journal (31) and the counterelement a nut (5, 15), which engages on a shoulder (43) of the sleeve (4).

8. Member according to claim 7, characterized in that the nut (15) is completely housed in the sleeve (4).

9. Member according to claims 7 or 8, characterized in that on its outer end the nut (5, 15) has an operating element, particularly an operating depression (17).

10. Member according to one of the preceding claims, characterized in that the strip (2, 12) substantially fills the interior of the helical spring (1). .

11. Member according to one of the preceding claim, characterized in that the strip (2, 12) is a wire rope comprising steel wires.

12. Member according to one of the preceding claims, characterized in that adjacent turns of the unbent helical spring (1) engage on one another.

13. Member according to one of the claims 4 to 12, characterized in that the slider (3) is prevented from rotating.

## Revendications

1. Organe élastiquement flexible à but orthopédique en forme de ressort hélicoïdal (1), caractérisé par le fait qu'un lien flexible, respectivement pliable (2, 12), est logé à l'intérieur du ressort hélicoïdal (1), ce lien étant agencé, au voisinage des extrémités du ressort hélicoïdal (1), de telle manière que, lors d'un allongement du ressort hélicoïdal (1) dû à un pliage de celui-ci, ce lien vienne en butée contre chaque extrémité du ressort hélicoïdal (1) ou contre une pièce (4, 24) solidaire de l'une d'elles, ce qui empêche la poursuite du pliage du ressort hélicoïdal (1), la distance entre les éléments en position de butée étant réglable.

2. Organe suivant la revendication 1, caractérisé par le fait que le lien (2, 12) est lié, à une de ses extrémités (19), au ressort hélicoïdal (1) ou à une pièce de liaison avec celui-ci (24).

3. Organe suivant les revendications 1 ou 2, caractérisé par le fait que le lien (2, 12) est lié, à une de ses extrémités (13, 19), à une pièce (4, 24) dont la dimension transversale est supérieure à la dimension transversale de l'espace intérieur du ressort hélicoïdal (1).

4. Organe suivant l'une des revendications précédentes, caractérisé par le fait que le lien (2, 12) est fixé, à une de ses extrémités, à un patin (3) qui, dans la zone de l'extrémité du ressort hélicoïdal concernée, est coulissant, la course de glissement étant réglable.

5. Organe suivant la revendication 4, caractérisé par le fait que la patin (3) est monté coulissant dans un manchon (4) relié au ressort hélicoïdal (1).

6. Organe suivant la revendication 5, caractérisé par le fait que le patin (3) présente un filetage qui coopère avec un taraudage d'un contre-élément réciproque qui, à son tour, coopère avec une surface de butée du manchon (4).

7. Organe suivant l'une des revendications 4 à 6, caractérisé par le fait que le patin (3) présente un tenon fileté (31), le contre-élément étant constitué par un écrou (5, 15) qui vient en appui sur un épaulement (43) du manchon (4).

8. Organe suivant la revendication 7, caractérisé par le fait que l'écrou (15) est entièrement logé dans le manchon (4).

9. Organe suivant la revendication 7 ou 8, caractérisé par le fait que l'écrou (5, 15) présente, à son extrémité extérieure, un élément de commande, en particulier une creusure de commande (17).

10. Organe suivant l'une des revendications précédentes, caractérisé par le fait que le lien (2, 12) remplit sensiblement tout l'intérieur du ressort hélicoïdal (1).

11. Organe suivant l'une des revendications précédentes, caractérisé par le fait que le lien (2, 12) est constitué par un câble formé de fils d'acier.

12. Organe suivant l'une des revendications précédentes, caractérisé par le fait que les spires voisines du ressort hélicoïdal (1), à l'état non plié, sont en contact les unes avec les autres.

13. Organe suivant l'une des revendications 4 à 12, caractérisé par le fait que le patin (3) est monté de façon à ne pas pouvoir tourner sur lui-même.

# Fig.1

# Fig.2

# Fig.3

# Fig.4

FIG. 5